# EUROPEAN PATENT APPLICATION

(11) **EP 1 897 533 A1**
(43) Date of publication of application: **12.03.2008**
(21) Application number: 06120400.4
(22) Date of filing: 08.09.2006
(51) Int. Cl.: A61K 8/60, A61Q 19/08, A61Q 19/00, A61Q 17/00

(54) **Use of 1,6-Bis [3-(3-carboxymethylphenyl)-4-(2-alpha -D-mannopyranosyl-oxy)-phenyl] hexane for the preparation of cosmetic compositions**

(71) Applicant: Revotar Biopharmaceuticals AG, 16761 Henningsdorf (DE)
(72) Inventor: Aydt, Ewald, 14163 Berlin (DE); Bock, Daniel, 30161 Hannover (DE); Vollhardt, Karin, 16761 Henningsdorf (DE); Wolff, Gerhard, 16548 Glienicke-Nordbahn (DE)
(74) Representative: Isenbruck, Günter

(57) **Abstract**

A compound of formula (I) or a polymorphic form thereof can be used for the preparation of a cosmetic or dermatological composition.

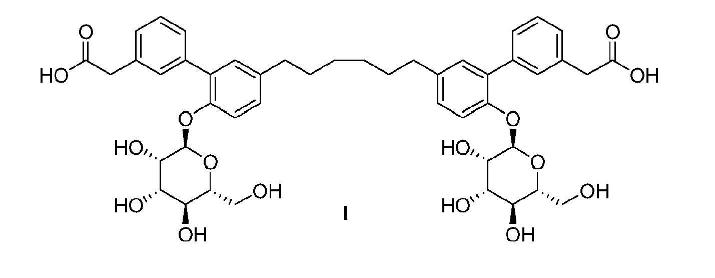

## Description

The present invention relates to the use of 1,6-Bis [3-(3-carboxymethylphenyl)-4-(2-αα-D-mannopyranosyloxy)-phenyl] hexane for the preparations of cosmetic and dermatological compositions. The invention furthermore relates to the treatment, diagnosis, and prophylaxis of micro-inflammatory conditions.

Cell-adhesion molecule-mediated functions are part of a complex cascade leading to the migration of circulating white blood cells (leukocytes) from the blood stream into the surrounding tissue (extravasation). Physiologically, leukocyte extravasation is of critical importance for homeostasis and immuno-surveillance of living beings including humans. Lymphocytes for example, are constitutively leaving the blood stream into lymphatic tissues in order to patrol for harmful antigens. Under pathological circumstances however, e.g. local or systemic inflammation and/or injury of the vascular system, this fundamental process is dys-regulated, at least in part, due to an increased surface expression of the adhesion molecules E- and P-selectin. Consequently, the excessive leukocyte extravasation leads to a pathological cellular infiltrate with subsequent tissue damage in several clinically relevant settings.

Disease states such as acute lung injury (ALI), acute respiratory distress syndrome (ARDS), asthma bronchiale (asthma), chronic obstructive pulmonary disease (COPD), psoriasis, rheumatoid arthritis, and sepsis are all associated with tissue inflammation induced and perpetuated by pathologically activated leukocytes infiltrating the respective tissue.

In addition, exaggerated leukocyte infiltration contributes to the pathogenesis of ischemic-reperfusion injury (IRI) associated with organ transplantation, cardiopulmonary bypass or percutaneous transluminal angioplasty. To extravasate, leukocytes must bind to the vascular endothelium in order to finally transmigrate into the surrounding tissue. Therefore, leukocytes have to attach and roll on the endothelium (tethering and rolling). This primary event in extravasation is mediated by the selectin family of cell-adhesion molecules. In addition to directly binding to the endothelium, leukocytes can adhere to other leukocytes, leukocyte-particles, platelets or platelet-derived particles that are already attached to the endothelium.

In addition to rolling and attachment mediated by the interaction of leukocytes and selectins, binding to selectins may also results in signal transduction [E. Crockett-Torabi, J. Leukocyte Biol., 1998, 63, 1-14]. It was shown that small molecules that bind to selectins can induce signal transduction as well [B. Brenner et al., PNAS 1996, 93, 15376-15381].
Furthermore, selectins are also involved in leukocyte retention in lung [D. Bock et al., Curr. Respir. Med. Rev., 2006, 2, 339-354].

The selectin family of adhesion molecules is comprised of three structurally related calcium-dependent carbohydrate binding cell surface proteins, E-, P- and L-selectin. E-selectin is expressed on inflamed endothelium, P-selectin is expressed on inflamed endothelium as well as on platelets and L-selectin is expressed on leukocytes. Selectins are composed of an amino terminal lectin domain, an epidermal growth factor (EGF)-like domain, a variable number of complement receptor-related repeats, a hydrophobic transmembrane domain and a C-terminal cytoplasmic domain. The binding interactions leading to the adhesion of the leukocytes are supposed to be mediated by contact of the lectin domain of the selectins and various carbohydrate ligands on the surface of the leukocytes.

All three selectins can bind with low affinity to the carbohydrate sialyl Lewis X (sLe^{X}), a glycosyl moiety present on the surface of most leukocytes. A structurally related glycosyl moiety, sialyl Lewis a (sLe^{a}), is predominantly found on the surface of cancer cells [K. Okazaki et al., J. Surg. Res., 1998, 78(1). 78-84; R. P. McEver et al., Glycoconjugate Journal, 1997, 14(5), 585-591].

In case of P-selectin, a distinct high affinity glycoprotein ligand has been described [R.P. McEver, R.D. Cummings, J.Clin.Invest., 1997, 100, 485-492], the so-called P-selectin glycoprotein ligand-1 (PSGL-1), which contributes to a high affinity selectin binding by its sLe^{X} moiety as well as by parts of its peptide components, in particular sulphated tyrosine residues [R.P. McEver, Ernst Schering Res. Found. Workshop, 2004, 44, 137-147]. PSGL-1 is one of the most important selectin ligands binding with highest affinity to P-selectin, but it also binds to E- and L-selectin [G. Constantin; Drug News Perspect; 2004; 17(9); 579-586]. It is a homodimeric sialomucin predominantly expressed on leukocytes.

In inflammatory diseases, dys-regulated extravasation is, at least in part, mediated due to an increased cell surface expression of E- and P-selectin. In contrast to their low basal expression, E- and P-selectin expression is upregulated during inflammation, leading to a substantial recruitment of leukocytes into the inflamed tissue. Although selectin-mediated cell adhesion is required for fighting infection, there are various situations in which such cell adhesion is undesirable or excessive, resulting in severe tissue damage instead of repair. In the case of many acute as well as chronic inflammatory disorders (e.g., asthma, COPD, psoriasis, etc.), an association between infiltration of activated leukocytes into the tissue simultaneously with a marked elevation of tissue expression of corresponding adhesion molecules, particularly E- and P-selectin, has been demonstrated [Muller et al., J. Pathol., 2002, 198(2), 270-275; Di Stefano et al., Am. J. Respir. Crit. Care. Med., 1994, 149(3) 803-810; Terajima et al., Arch. Dermatol. Res., 1998, 290, 246-252].

Leukocyte infiltration may also play a role in inflammatory symptoms in the course of transplant and graft rejection. Also the process of blood clotting is further promoted by leukocyte-leukocyte and leukocyte-platelet binding, which occurs because leukocytes possess both L-selectin and its corresponding ligand P-glycoprotein ligand-1 (PSGL-1) and can thus interact with themselves via PSGL-1, and they can also bind to platelets which carry P-selectin. In addition, selectins are involved in micro-inflammatory processes causing ageing of the skin [P. U. Giacomoni et al., Micron 2004, 35, 179-184].

The signs of ageing of the skin resulting from the effects on the skin of intrinsic and extrinsic factors are defined by the appearance of wrinkles and fine lines, by the yellowing of the skin which develops a wizened appearance along with the appearance of pigmentation blemishes, by a change in the thickness of the skin, generally resulting in a thickening of the stratum corneum and of the epidermis and a thinning of the dermis, by disorganization of the elastin and collagen fibers which causes a loss of elasticity, of suppleness and of firmness, and by the appearance of telnagiectasia.

Some of these signs are more particularly associated with intrinsic or physiological ageing, that is so to say with "normal" ageing associated with age, whereas others are more specific to extrinsic ageing, that is so to say ageing caused by the environment in general; such ageing is more particularly photo-ageing due to exposure to the sun, to light or to any other radiation. Other factors causing ageing of the skin are atmospheric pollution, wounds, infections, traumatisms, anoxia, cigarette smoke, hormonal status, neuropeptides, electromagnetic fields, gravity, lifestyle (e.g. excessive consumption of alcohol), repetitive facial expressions, sleeping positions, and psychological stressors.

The changes in the skin which occur due to intrinsic ageing are the consequence of a genetically programmed sequence involving endogenous factors. This intrinsic ageing in particular causes slowing down of the regeneration of skin cells, which is reflected essentially in the appearance of clinical damage such as a reduction of the subcutaneous adipose tissue and the appearance of fine lines or small wrinkles, and in histopathological changes such as an increase in the number and thickness of the elastic fibers, a loss of vertical fibers from the elastic tissue membrane and the presence of large irregular fibroblasts in the cells of this elastic tissue.

In contrast, extrinsic ageing results in clinical damage such as thick wrinkles and the formation of flabby and weather-beaten skin, and in histopathological changes such as an excessive accumulation of elastic substance in the upper dermis and degeneration of the collagen fibers.

Intrinsic and extrinsic factors of ageing of the skin share common mechanisms [P. U. Giacomoni et al., Biogerontology 2004, 2, 219-229]. These factors trigger a process leading to the accumulation of damages in the skin resulting in skin ageing since the expression of cell adhesion molecules provokes recruitment and diapedesis of circulating immune cells, which digest the extracellular matrix (ECM) by secreting collagenases, myeloperoxidases and reactive oxygen species.

The activation of these lytic processes provokes random damage of these resident cells, which in turn secrete prostaglandins and leukotrienes. These signaling molecules induce the degranulation of resident mast cells which release the autacoid histamine and the cytokine TNFalpha thus activating endothelial cells lining adjacent capillaries which release P-selectin and the synthesis of cell adhesion molecules such as E-selectin and ICAM-1. This closes a self-maintained micro-inflammatory cycle, which results in the accumulation of ECM damage, i.e. skin ageing.

There is a strong cosmetic and dermatological need for novel compounds for the treatment or prophylaxis of skin ageing. Various cosmetic and dermatological compositions (including for skin care) intended inter alia to prevent or treat ageing of the skin are known. Retinoic acid and derivatives thereof have been described as anti-ageing agents in skin care, cosmetic, or dermatological compositions, in particular in US 4603146. α-Hydroxy acids such as lactic acid, glycolic or alternatively citric acid are also known for this same application, these acids having been described in numerous patents and publications (e.g. EP-A-413528) and introduced into numerous skin care, cosmetic, or dermatological compositions on the market. Aromatic orthohydroxy acids such as salicylic acid have also been proposed (e.g. WO 93/10756 and WO 93/10755).

All of these compounds act against ageing of the skin by desquamation, that is to say removal of the dead cells at the surface of the stratum corneum. This desquamation is also referred to as a keratolytic property. However, these compounds also have side effects, consisting of stinging and redness, which the user finds unpleasant. Thus, there remains a need for anti-ageing agents which are at least as effective as the known compounds, but do not exhibit their drawbacks. Unlike the established strategies to treat or prevent skin ageing, modulating the selectin function is a novel concept intervening the micro-inflammation cascade at a very early stage and treating and preventing intrinsic and extrinsic skin ageing according to the present inventions represents a strategy without the drawbacks known from other strategies.

1,6-Bis [3-(3-carboxymethylphenyl)-4-(2-α-D-mannopyranosyloxy)-phenyl] hexane (compound of formula (I)) is described, for example, in US 5919768 and US 5712387 and EP-A 0840606, which are incorporated herein by reference and whose contents are explicitly part of the present disclosure. The preferred stereoisomeric form of compound of formula (I) is identical with the form as shown below.

The compound of formula (I) has valuable pharmacological properties. It acts as panselectin antagonist and therefore inhibits leukocyte extravasation. Since leukocyte extravasation is a key step in the pathogenesis of most inflammatory disorders or conditions the compound of formula (I) offers the opportunity to be developed in a variety of inflammatory indications.

The compound of formula (I) can therefore be used for the prophylaxis, treatment, and diagnosis of inflammatory disorders covering a variety of medical indications.

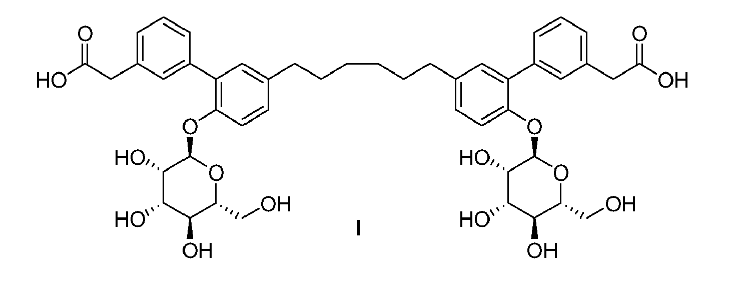

The compound of formula (I) and its physiologically tolerable salts are suitable as active active pharmaceutical ingredients (API) for the prevention, treatment, and diagnosis of various inflammatory diseases.

Furthermore the compound of formula (I) may be used for the treatment or prevention of ageing of the skin caused by intrinsic or extrinsic factors such as age, environment in general; more particularly photo-ageing due to exposure to the sun, to light or to any other radiation, atmospheric pollution, wounds, infections, traumatisms, anoxia, cigarette smoke, hormonal status, neuropeptides, electromagnetic fields, and psychological stressors.

The compound of formula (I) occurs in different polymorphic or pseudo-polymorphic forms, whereby herein the term "FORM" is used to describe both polymorphs and pseudopolymorphs including solvates and hydrates. The different FORMS, which can be prepared specifically by adjustment of the reaction conditions and/or of the crystallization conditions, differ in their structural and physicochemical properties as well as in their pharmacological properties, e.g. bioavailability.

The FORMS differ, for example, in their solubility, rate of dissolution or the behavior during pharmaceutical processing and allow the production of pharmaceutical compositions having different property profiles starting from a single parent compound. FORM 1 of compound of formula (I) is characterized by a strong X-ray reflection angle 2Theta (in °) in the X-ray diffraction diagram using Cu K_{α1} radiation at 2Theta = 4.8 . FORM 2 of compound of formula (I) has X-ray reflections diffraction angles 2Theta (in °) in the X-ray diffraction diagram using Cu K_{α1} radiation with strong intensity at 2Theta =5.3 and 5.6 and 17.4° and with medium intensity at 2Theta = 15.1°.
FORM 3 of compound of formula (I) has X-ray reflections diffraction angles 2Theta (in °) in the X-ray diffraction diagram using Cu K_{α1} radiation with strong intensity at 2Theta = 5.3 and 5.6° and with medium intensity at 2Theta = 4.2, 4.3 and 4.8°

The use of the active ingredients used according to the invention or of cosmetic or topical dermatological compositions with an effective content of active ingredient used according to the invention surprisingly enables effective treatment, but also prophylaxis of skin ageing caused by extrinsic and intrinsic factors.

The invention particularly relates to the use of a compound of formula (I) or a stereoisomeric or polymorphic form thereof for the preparation of a cosmetic or dermatological composition.

The amount used of compound of formula (I) or a stereoisomeric or polymorphic form thereof corresponds to the amount required to obtain the desired result using the cosmetic or dermatological compositions. One skilled in this art is capable of evaluating this effective amount, which depends on the derivative used, the individual on whom it is applied, and the time of this application. To provide an order of magnitude, in the cosmetic or dermatological compositions according to the invention, the compound of formula (I) or a stereoisomeric or polymorphic form thereof may be administered in an amount representing from 0.001% to 40% by weight, preferentially 0.005% to 30% by weight and more preferentially from 0.01 % to 20% by weight.

A further aspect covers cosmetic compositions comprising a compound of formula (I) or a stereoisomeric or polymorphic form thereof and at least one cosmetically tolerable component, e.g. a cosmetically tolerable component for skin applications.

The amounts of the various components of the physiological medium of the cosmetic composition according to the invention are those generally included in the fields under consideration. When the cosmetic composition is an emulsion, the proportion of the fatty phase may range from 2% to 80% by weight and preferably from 5% to 50% by weight relative to the total weight of the cosmetic composition.

The aqueous phase is adjusted as a function of the content, in the fatty phase, of compound of formula (I) or stereoisomeric or polymorph from thereof, of alcohol and nonionic surfactant and also that of the optional additional ingredients, to obtain 100% by weight. In practice, the aqueous phase represents from 5% to 90% by weight. The fatty phase may contain fatty or oily compounds, which are liquid at room temperature (25°C.), generally known as oils, waxes and pasty or semi-solid products. These oils may be mutually compatible and may form a macroscopically homogeneous liquid fatty phase. The aqueous phase contains water and optionally a water-miscible ingredient, for instance polyols such as propylene glycol, glycerol or sorbitol.

In particular, the cosmetic composition often contains one or more components such as nonionic surfactants. The nonionic surfactant or the mixture of nonionic surfactants with a hydrophilic-lipophilic balance (HLB) of greater than 10 is (are) preferably used in an amount sufficient to dissolve with the compound of formula (I) or a stereoisomeric or polymorphic form thereof. In practice, this nonionic surfactant or mixture of nonionic surfactants may be included in the compositions of the invention in a concentration ranging from 0.01% to 10% by weight, preferentially from 0.05% to 5% by weight and more preferentially from 0.1 % to 2% by weight.

More specifically, cosmetic compositions of the present invention contain nonionic surfactants having a HLB of greater than 10 [Griffin, W. C., J. Soc. Cosmet. Chem.,1949, 1, 311, and 1954, 5, 249] and which may be up to 20. These are compounds that are well known per se [Handbook of Surfactants by M. R. Porter, published by Blackie & Son (Glasgow and London), 1991, pp. 116-178]. Thus, they may be selected especially from among polyethoxylated, polypropoxylated or polyglycerolated fatty acids, (C1-C20) alkylphenols, alpha-diols and alcohols, which are preferably hydrogenated, with a fatty chain containing, for example, from 8 to 22 carbon atoms, the mean number of ethylene oxide or propylene oxide structural units optionally ranging especially from 3.5 to 200 (for example from 5 to 100) and the number of glycerol groups optionally ranging especially from 2 to 100 (for example from 3 to 50), and mixtures thereof. Also exemplary are copolymers of ethylene oxide and propylene oxide, condensates of ethylene oxide and propylene oxide on fatty alcohols; polyethoxylated fatty amides and preferably those containing on average from 3.5 to 200 mol of propylene oxide and/or ethylene oxide; polyglycerolated fatty amides and preferably those containing on average from 1.5 to 40; ethoxylated fatty acid esters of sorbitan especially containing from 2 to 30 mol on average of ethylene oxide and a fatty chain especially containing from 8 to 22 (for example from 12 to 18) carbon atoms; fatty acid esters of sucrose; fatty acid esters of polyethylene glycol; (C6-C24)alkylpolyglycosides; N-(C6-C24) alkylglucamine derivatives; amine oxides such as(C10-C14) alkylamine oxides; and mixtures thereof.

In the cosmetic compositions an alcohol may be used alone or as a mixture and is selected from among C1-C4 alcohols such as ethanol or isopropanol, and mixtures thereof. The C1-C4 alcohol is preferably included in an amount sufficient to dissolve with the nonionic surfactant the compound of formula (I) or a stereoisomeric or polymorphic form thereof. In practice, the C1-C4 alcohol or the mixture of C1-C4 alcohols may be included in the cosmetic compositions of the invention in a concentration ranging from 2% to 80% of the total weight of the composition, preferentially from 10% to 70% and more preferentially from 20% to 60% by weight relative to the total weight of the cosmetic composition. The cosmetic compositions of the invention are preferentially for cosmetic applications and in particular for topical application to the skin.

Thus, the cosmetic composition should contain a non-toxic physiologically acceptable medium that can be applied to human skin. For a topical application to the skin, the cosmetic composition may be in the form of a solution, a suspension, an emulsion or a dispersion of more or less fluid consistency and especially liquid or semi-liquid consistency, obtained by dispersing a fatty phase in an aqueous phase (O/W) or, conversely, (W/O), or alternatively a gel. A cosmetic composition in the form of a mousse or in the form of a spray or an aerosol then comprising a pressurized propellant may also be provided. Also the compositions may be in the form of a haircare lotion, a shampoo or hair conditioner, a liquid or solid soap, a treating mask, or a foaming cream or gel for cleansing the hair. They may also be in the form of a hair dye or hair mascara.

The cosmetic compositions of the invention may also comprise one or more other ingredients usually employed in the fields under consideration, selected from among formulation additives, for instance aqueous-phase or oily-phase thickeners or gelling agents, dyestuffs that are soluble in the medium of the cosmetic composition, solid particles such as mineral or organic fillers or pigments in the form of microparticles or nanoparticles, preservatives, fragrances, hydrotopes or electrolytes, neutralizers (acidifying or basifying agents), propellants, anionic, cationic or amphoteric surfactants, polymers, in particular water-soluble or water-dispersible anionic, nonionic, cationic or amphoteric film-forming polymers, mineral or organic salts, chelating agents; mixtures thereof.

These additives may be present in the cosmetic composition in the amounts generally employed in cosmetics, and especially in a proportion of from 0.01% to 50% and preferably from 0.1 % to 20%, for example from 0.1 % to 10%, of the total weight of the cosmetic composition. Oils that may be included according to the invention may be made of oils of mineral origin (liquid petroleum jelly or hydrogenated isoparaffin), oils of plant origin (liquid fraction of shea butter, sunflower oil, apricot oil, soybean oil, fatty alcohol or fatty acid), oils of animal origin (perhydrosqualene), synthetic oils (fatty acid esters or purcellin oil), silicone oils (linear or cyclic polydimethylsiloxanes, and phenyl trimethicones) and fluoro oils (perfluoropolyethers).

Waxes that may be mentioned include silicone waxes, beeswax, candelilla wax, rice bran wax, carnauba wax, paraffin wax or polyethylene wax. When the cosmetic composition is in emulsion form, it also contains one or more emulsifiers and optionally one or more co-emulsifiers generally employed in cosmetics. Their nature also depends on the sense of the emulsion. In practice, the emulsifier and, optionally, the co-emulsifier are present in the cosmetic composition in a proportion ranging from 0.1% to 30% by weight, preferably from 0.5% to 20% by weight and better still from 1% to 8% by weight. The emulsion may also contain lipid vesicles and especially liposomes. As thickeners or gelling agents that may be included according to the invention, mention may be made of thickening or gelling polymers, for instance crosslinked polyacrylic acids, associative polymers and non-polymeric thickeners or gelling agents, for instance modified or unmodified clays, amides and metal salts of fatty acids.

The cosmetic compositions may be used to inhibit the micro-inflammatory cycle. Thus, the present invention also relates to cosmetic compositions comprising a compound of formula (I) or a stereoisomeric or polymorphic form thereof that is used for the cosmetic treatment or cosmetic prophylaxis of micro-inflammatory conditions.

Cosmetic compositions comprising a compound of formula (I) or a stereoisomeric or polymorphic form thereof that is used for the cosmetic treatment or cosmetic prophylaxis of skin ageing caused by intrinsic factors are also subject of the present invention. Intrinsic factors responsible for skin ageing are genetically programmed determinants including age, hormonal status, and psychological factors.

Beside cosmetically inactive ingredients the cosmetic compositions of the present invention may also comprise one or more cosmetically active ingredients with beneficial action on the skin. Thus, the present invention relates to cosmetic compositions comprising a compound of formula (I) or a stereoisomeric or polymorphic form thereof and at least one further cosmetically active ingredient.

As cosmetically/pharmaceutically active agents with beneficial action the following are cited: of active agents selected from among: UV-blocking agents, such as sunscreens; vitamins (A, C or E) and derivatives thereof (retinyl palmitate, tocopheryl acetate or tocopheryl palmitate); ceramides; proteins and protein hydrolysates, peptides and amino acids; urea and allantoin; sugars and sugar derivatives, for instance reduced or oxidized sugars; extracts of plant origin (those from Iridacea plants or from soybean) or of bacterial origin; hydroxy acids, in particular hydroxycarboxylic acids or ketocarboxylic acids (fruit acid, salicylic acid) and esters thereof, for instance 5-n-octanoylsalicylic acid; diazoxide, spiroxazone, or phospholipids, for instance lecithin; additional active compounds , especially: nicotinic acid esters, among which especially are tocopheryl nicotinate, benzyl nicotinate and C1-C22 alkyl nicotinates such as methyl or hexyl nicotinate; pyrimidine derivatives, for instance 2,4-diamino-6-piperidinopyrimidine 3-oxide or "Minoxidil" described in US4139619 and US4592812; pyrimidine 3-oxide derivatives, for instance those described in WO 92/01437 or WO 96/09048, and especially "Aminexil" or 2,4-diaminopyrimidine 3-N-oxide; anti-androgens, for instance steroidal or non-steroidal inhibitors of 5-[alpha]-reductase, such as finasteride and the compounds described in US5516779, cyprosterone acetate, azeleic acid, its salts and its derivatives and the compounds described in US5480913, flutamide and the compounds described in US5411981, US5565467 and US4910226, prostaglandins and analogues thereof, for instance latanoprost; anti-bacterial, anti-fungal or anti-dandruff agents, for instance selenium derivatives, ketoconazole, octopirox, triclocarban, triclosan, zinc pyrithione, itraconazole, asiatic acid, hinokitiol, mipirocine, tetracyclines, especially erythromycin and the compounds described in EP-A 0,680,745, clinycine hydrochloride, benzoyl peroxide or benzyl peroxide and minocycline; calcium-channel antagonists and potassium-channel agonists; hormones; steroidal anti-inflammatory agents, for instance glucocorticoids, corticosteroids (for example: hydrocortisone) and non-steroidal anti-inflammatory agents, for instance glycyrrhetinic acid and [alpha]-bisabolol, benzydamine and the compounds described in EP-0,770,399, WO 94/06434;

retinoid RXR receptor agonists and retinoid antagonists; free-radical scavengers and antioxidants, for instance butylhydroxyanisole or butylhydroxytoluene; anti-seborrhoeic agents; anti-parasitic agents; anti-viral agents; anti-pruriginous agents; carotenoids, for instance [beta]-carotene; lactones and the corresponding salts thereof; essential fatty acids, for instance linoleic acid, eicosatetraenoic acid, linolenic acid and eicosatrienoic acid, or esters and amides thereof; essential oils; phenols and polyphenols, for instance flavonoids; and mixtures thereof. This additional active agent may be for example in an amount of from 0.001% to 10% by weight, preferably from 0.1% to 5% and better still from 0.5% to 3% by weight.

Cosmetic compositions comprising a compound of formula (I) or a stereoisomeric or polymorphic form thereof and at least one further cosmetically active ingredient characterized in that it is used for the cosmetic treatment or cosmetic prophylaxis of micro-inflammatory conditions are also subject of the invention.

In particular the present invention relates to cosmetic compositions comprising a compound of formula (I) or a stereoisomeric or polymorphic form thereof and at least one further cosmetically active ingredient characterized in that it is used for the cosmetic treatment or cosmetic prophylaxis of skin ageing caused by intrinsic factors.

Dermatological compositions comprising a compound of formula (I) or a stereoisomeric or polymorphic form thereof and at least one dermatologically tolerable component, e.g. a dermatologically tolerable component for skin applications, are also subject of the invention.
Dermatologically tolerable components that can be used for the dermatological compositions described here are identical to the cosmetically tolerable components as defined in this invention.

A further embodiment of this invention are dermatological compositions comprising a compound of formula (I) or a stereoisomeric or polymorphic form thereof that is used for the dermatological treatment, dermatological diagnosis or dermatological prophylaxis of micro-inflammatory conditions.

In particular the invention covers dermatological compositions comprising a compound of formula (I) or a stereoisomeric or polymorphic form thereof that is used for the dermatological treatment, dermatological diagnosis or dermatological prophylaxis of itching and skin ageing caused by extrinsic factors. Extrinsic factors include environmental factors in general; more particularly photo-ageing due to exposure to the sun, to light or to any other radiation, atmospheric pollution, wounds, infections, traumatisms, anoxia, cigarette smoke, hormonal status as response to external factors, neuropeptides, electromagnetic fields, gravity, lifestyle (e.g. excessive consumption of alcohol), repetitive facial expressions, sleeping positions, and psychological stressors.

In addition to dermatologically inactive ingredients the dermatological compositions may also comprise dermatologically or pharmaceutically active ingredients. Thus, the present invention also relates to dermatological compositions comprising a compound of formula (I) or a stereoisomeric or polymorphic form thereof and at least one further dermatologically or pharmaceutically active ingredient. The dermatologically or pharmaceutically active ingredients that can be used for the dermatological compositions described herein are defined as the cosmetically active ingredients defined above. Dermatologically or pharmaceutically active ingredients can be identical to the cosmetically active ingredients as defined in this invention.

Another subject of the present invention are dermatological compositions comprising a compound of formula (I) or a stereoisomeric or polymorphic form thereof and at least one further dermatologically or pharmaceutically active ingredient characterized in that is used for the dermatological treatment, dermatological diagnosis or dermatological prophylaxis of micro-inflammatory conditions.

In particular, the present invention relates to dermatological compositions comprising a compound of formula (I) or a stereoisomeric or polymorphic form thereof and at least one further dermatologically or pharmaceutically active ingredient characterized in that it is used for the dermatological treatment, dermatological diagnosis or dermatological prophylaxis of itching and skin ageing caused by extrinsic factors.

Ageing of the skin may also be caused by a combination of intrinsic and extrinsic factors. Therefore, the present invention also relates to dermatological compositions comprising a compound of formula (I) or a stereoisomeric or polymorphic form thereof and at least one further pharmaceutically or cosmetically active ingredient characterized in that it is used for the cosmetic and dermatological treatment and cosmetic and dermatological prophylaxis of skin ageing caused by a combination of intrinsic and extrinsic factors.

Another embodiment of this invention is a process for the preparation of a cosmetic composition by mixing a compound of formula (I) or a stereoisomeric or polymorphic form thereof, at least one cosmetically tolerable component and eventually further cosmetically active ingredients.

In particular, a process for the preparation of a cosmetic composition by mixing a compound of formula (I) or a stereoisomeric or polymorphic form thereof, at least one cosmetically tolerable component and eventually further cosmetically active ingredients, wherein the composition includes from 0.01% to 20% by weight of compound of formula (I) or a stereoisomeric or polymorphic form thereof, based on the total weight of the composition is subject of this invention.

A further aspect deals with a process for the preparation of a dermatological composition by mixing a compound of formula (I) or a stereoisomeric or polymorphic form thereof, at least one dermatologically tolerable component and eventually further pharmaceutically active ingredients.

In particular, a process for the preparation of a dermatological composition by mixing a compound of formula (I) or a stereoisomeric or polymorphic form thereof, at least one dermatologically tolerable component and eventually further pharmaceutically active ingredients, wherein the composition includes from 0.01% to 20% by weight of compound of formula (I) or a stereoisomeric or polymorphic form thereof, based on the total weight of the composition is subject of the present invention.

The cosmetic and dermatological compositions contain, for example, compound of formula (I) or a stereoisomeric or polymorphic form thereof and a cosmetically, or dermatologically tolerable component. The cosmetic and dermatological preparations can be manufactured using standard technologies, known to the person skilled in the art. For this, the compound of formula (I) or a stereoisomeric or polymorphic form thereof according to the invention are brought into a suitable dosage form together with one or more cosmetic or dermatological vehicles and/or inactive ingredients and, if the preparation of a combination preparation is desired, one or more other pharmaceutical, cosmetic, or dermatological active compounds having therapeutic, or prophylactic action, which can then be used as cosmetic or dermatological product.

Other features of the invention will become apparent in the course of the following descriptions of exemplary embodiments, which are given for illustration of the invention and are not intended to be limiting thereof.

### Examples

In the following examples the percentages are given by weight. The term "qs 100%" means that a sufficient amount of that ingredient is present to make the sum of the amounts for all ingredients equal 100%.

### Example 1

1400 g of a care cream having the following composition is prepared according to the methods known to the person skilled in the art by mixing of 0.7% of 1,6-Bis [3-(3-carboxymethylphenyl)-4-(2-α-D-mannopyranosyloxy)-phenyl] hexane, 0.75% of Span 65 ® (sorbitane tristearate, detergent, MerckSchuchardt OHG), 2.5% of liquid petrolatum, 1% of Sepigel 305 ® (Polyacrylamide/C13.14 Isoparaffin/Laureth-7 - SEPPIC, thickening and emulsifying agent), 3% of glyceryl monostearate, 2% of Myrj 52 ® (polyoxyethylene 40 stearate, emulsifier, Sigma-Aldrich), 4% ofketostearyl alcohol (50-50), 15% of perhydrosqualene, 3.5% of glycerol, 0.15% of preserving agent, 0.03% of disodium EDTA, and qs100% of demineralized water. The pH value of the formula is adjusted to 4.0.

### Example 2:

1570 g of a cream having the following composition is prepared according to the methods known to the person skilled in the art by mixing of 0.6% of Form 1 of 1,6-Bis [3-(3-carboxymethylphenyl)-4-(2-α-D-mannopyranosyloxy)-phenyl] hexane, 0.75% of Span 65 ® (sorbitane tristearate, detergent, MerckSchuchardtOHG), 2.5% of liquid petrolatum, 1% of Sepigel 305 ® (Polyacrylamide/C13.14 Isoparaffin/Laureth-7 - SEPPIC, thickening and emulsifying agent), 2% of Myrj 52 (Polyoxyethylene 40 stearate, emulsifier, Sigma-Aldrich), 5% of ketostearyl alcohol (50-50), 17% of perhydrosqualene, 3.5% of glycerol, 0.15% of preserving agent, 0.03% of disodium EDTA, 0.5% of triethanolamine, and qs100% of demineralized water. The pH value of the formula is adjusted to 4.5.

### Example 3:

2100 g of a gel having the following composition is prepared according to the methods known to the person skilled in the art by mixing 0.5% of Form 1 of 1,6-Bis [3-(3-carboxymethylphenyl)-4-(2-α-D-mannopyranosyloxy)-phenyl] hexane, 1.5% of Eusolex 232 ® (Phenylbenzimidazole sulfonic acid, UVB filter, Merck KGaA), 1.2% of Carbomer 934 P ® (thickening, suspending and stabilizing substance, Goodrich), 4% of glycerol, 0.2% of triethanolamine, 2% of propylene glycol, 0.6% of xanthan gum, and qs100% of demineralized water. The pH value of the formula is adjusted to 4.6.

### Example 4:

1750 g of a tonic lotion having the following composition is prepared according to the methods known to the person skilled in the art by mixing 1% of Form 2 of 1,6-Bis [3-(3-carboxymethylphenyl)-4-(2-α-D-mannopyranosyloxy)-phenyl] hexane, 0.4% of D pantehnol, 4% of glycerol, 3% ofpropylene glycol, 0.03% of disodium EDTA, 0.1% of methyl para-oxybenzoate, 0.6% ofUVINUL MS 40 ® (benzophenone-4, UVB filter, BASF), 0.2% of PEMULEN TR1® (polymeric emulsifier, Goodrich), 0.2% of triethanolamine, and qs100% of demineralized water.

### Example 5:

2200 g of a fluid having the following composition is prepared according to the methods known to the person skilled in the art by mixing 1.2% of 1,6-Bis [3-(3-carboxymethylphenyl)-4-(2-α-D-mannopyranosyloxy)-phenyl] hexane, 5% of glycerol, 0.06% of disodium EDTA, 0.4% of EUSOLEX 232 ® (Phenylbenzimidazole sulfonic acid, UVB filter, Merck KGaA), 0.2% of triethanolamine, 0.2% of SEPIGEL 305 ® (Polyacrylamide/C13.14 Isoparaffin/Laureth-7 - SEPPIC, thickening and emulsifying agent), 0.3% of Generol 122 E5 ® (emulsifier, Henkel), 0.7% of hydrogenated soya lecithin, 0.6% of CARBOPOL 980 ® (polymer, thickening, Goodrich), 0.15% of apricot kernel oil, and qs100% of demineralized water.

### Example 6:

1650 g of a microemulsion having the following composition is prepared according to the methods known to the person skilled in the art by mixing 1.0% of FORM 1 of 1,6-Bis [3-(3-carboxymethylphenyl)-4-(2-α-D-mannopyranosyloxy)-phenyl] hexane, 8% of Tagat® O2 V (surfactant, Goldschmidt GmbH), 12% of Synperonic® PE/L101 (Poloxamer 331, surfactant, Uniqema), 5% of glyceryl triacetate, and qs100% of propylene glycol/ 0.005 N hydrochloric acid (2:1). The pH value of the formula is adjusted to 4.0.

### Example 7:

By using the microemulsion as described in example 6 skin penetration studies of FORM 1 of 1,6-Bis [3-(3-carboxymethylphenyl)-4-(2-α-D-mannopyranosyloxy)-phenyl] hexane employing an isolated perfused porcine fore limb transdermal penetration assay using pig porcine is performed according to the methods known to the skilled person. The isolated perfused porcine fore limb is an assay system, which retains intact anatomy and skin, muscle, bloos vessel and bone morphology. The organ is kept vitalized by perfusion with a blood-based perfusion medium for up to 6h. This system allows monitoring of transdermal, resorption, penetration or permeation of pharmaceuticals and cosmetics. 3 application sites are choosen and surrounded by Leukosilk. On each of these areas 10µl of microemulsion are applied with a pipette and distributed with the pipette tip.

For three additional application sites, the cotton pads of three Hill Top chambers in total, are soaked and saturated with microemulsion and applied to skin. After 5h application time Hill Top chambers are removed and the unabsorbed microemulsion from the free sites is removed with a dry swab each. 20 Tesa strips are collected from each of the six applications sites. Afterwards skin biopsys are punched out from each application site. Swabs, strips, skin biopsys are analysed for content of FORM 1 of 1,6-Bis [3-(3-carboxymethylphenyl)-4-(2-α-D-mannopyranosyloxy)-phenyl] hexane with LC/MS. On average 5% of the applied amount of FORM 1 of 1,6-Bis [3-(3-carboxymethylphenyl)-4-(2-α-D-mannopyranosyloxy)-phenyl] hexane in the microemulsion as described in example 6 penetrates into skin. No skin ageing is observed.

### Example 8:

By using the microemulsion as described in example 6 skin penetration studies of FORM 1 of 1,6-Bis [3-(3-carboxymethylphenyl)-4-(2-α-D-mannopyranosyloxy)-phenyl] hexane employing a Franz diffusion cell penetration assay using porcine skin is performed according to the methods known to the skilled person. Porcine skin samples (6 in total, 2 from each animal, 3 female animals in total), 1000µm thick, with the stratum corneum facing up are placed onto the lower part of a Franz diffusion cell. The cell is rinsed with medium tempered to 37°C to prevent the skin from drying out and the system is equilibrated for 30 minutes. 10.4µl of microemulsion as described in example 6 is applied to the dry upper side of the epidermis using a pipette and the microemulsion is distributed with an inoculation loop. Medium is collected every hour.

After 8h the experiment is finished. The skin is punched out and the surface of the skin is swabed to removed excess microemulsion. The dry surface is stripped 20 times using Tesafilm. Swabs, strips, medium and skin samples are analysed for content of FORM 1 of 1,6-Bis [3-(3-carboxymethylphenyl)-4-(2-α-D-mannopyranosyloxy)-phenyl] hexane by LC/MS. On average 4% of the applied amount of FORM 1 of 1,6-Bis [3-(3-carboxymethylphenyl)-4-(2-α-D-mannopyranosyloxy)-phenyl] hexane in the microemulsion as described in example 6 penetrates into skin. No skin ageing is observed.

## Claims

1. Use of a compound of formula (I) or a stereoisomeric or polymorphic form thereof for the preparation of a cosmetic or dermatological composition.

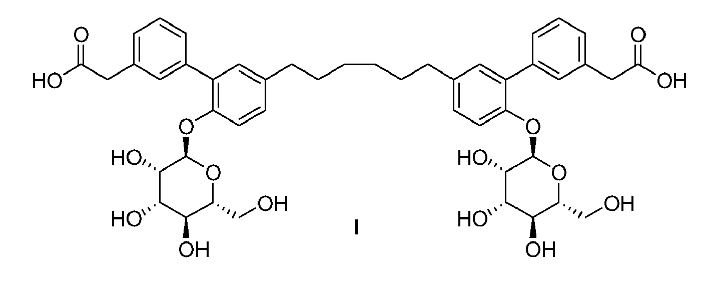

2. Cosmetic compositions comprising a compound of formula (I) or a stereoisomeric or polymorphic form thereof and at least one cosmetically tolerable component.

3. Cosmetic compositions according to claim 2, comprising a compound of formula (I) or a stereoisomeric or polymorphic form thereof and at least one cosmetically tolerable component for skin applications.

4. Cosmetic compositions according to one of the claims 2 or 3, comprising a compound of formula (I) or a stereoisomeric or polymorphic form thereof that is used for the cosmetic treatment or cosmetic prophylaxis of micro-inflammatory conditions.

5. Cosmetic compositions according to one of the claims 2 or 3, comprising a compound of formula (I) or a stereoisomeric or polymorphic form thereof that is used for the cosmetic treatment or cosmetic prophylaxis of skin ageing caused by intrinsic factors.

6. Cosmetic compositions according to one of the claims 2 or 5, comprising a compound of formula (I) or a stereoisomeric or polymorphic form thereof and at least one further cosmetically active ingredient.

7. Cosmetic compositions according to one of the claims 2 or 6, comprising a compound of formula (I) or a stereoisomeric or polymorphic form thereof and at least one further cosmetically active ingredient **characterized in that** it is used for the cosmetic treatment or cosmetic prophylaxis of micro-inflammatory conditions.

8. Cosmetic compositions according to one of the claims 2 or 6, comprising a compound of formula (I) or a stereoisomeric or polymorphic form thereof and at least one further cosmetically active ingredient **characterized in that** it is used for the cosmetic treatment or cosmetic prophylaxis of skin ageing caused by intrinsic factors.

9. Dermatological compositions comprising a compound of formula (I) or a stereoisomeric or polymorphic form thereof and at least one dermatologically tolerable component.

10. Dermatological compositions according to claim 9, comprising a compound of formula (I) or a stereoisomeric or polymorphic form thereof and at least on dermatologically tolerable component for skin application.

11. Dermatological compositions according to one of the claims 9 or 10, comprising a compound of formula (I) or a stereoisomeric or polymorphic form thereof that is used for the dermatological treatment, dermatological diagnosis or dermatological prophylaxis of micro-inflammatory conditions.

12. Dermatological compositions according to on of the claims 9 or 10, comprising a compound of formula (I) or a stereoisomeric or polymorphic form thereof that is used for the dermatological treatment, dermatological diagnosis or dermatological prophylaxis of itching and skin ageing caused by extrinsic factors.

13. Dermatological compositions according to one of the claims 9 or 12, comprising a compound of formula (I) or a stereoisomeric or polymorphic form thereof and at least one further dermatologically or pharmaceutically active ingredient.

14. Dermatological compositions according to one of the claims 9 or 13, comprising a compound of formula (I) or a stereoisomeric or polymorphic form thereof and at least one further dermatologically or pharmaceutically active ingredient **characterized in that** is used for the dermatological treatment, dermatological diagnosis or dermatological prophylaxis of micro-inflammatory conditions.

15. Dermatological compositions according to one of the claims 9 or 13, comprising a compound of formula (I) or a stereoisomeric or polymorphic form thereof and at least one further dermatologically or pharmaceutically active ingredient **characterized in that** it is used for the dermatological treatment, dermatological diagnosis or dermatological prophylaxis of itching and skin ageing caused by extrinsic factors.

16. Dermatological compositions according to claims 9 to 13, comprising a compound of formula (I) or a stereoisomeric or polymorphic form thereof and at least one further pharmaceutically or cosmetically active ingredient **characterized in that** it is used for the cosmetic and dermatological treatment and cosmetic and dermatological prophylaxis of skin ageing caused by a combination of intrinsic and extrinsic factors.

17. Process for the preparation of a cosmetic composition according to claims 2 to 8 by mixing a compound of formula (I) or a stereoisomeric or polymorphic form thereof, at least one cosmetically tolerable component and eventually further cosmetically active ingredients.

18. Process for the preparation of a cosmetic composition according to claim 17 by mixing a compound of formula (I) or a stereoisomeric or polymorphic form thereof, at least one cosmetically tolerable component and eventually further cosmetically active ingredients, wherein the composition includes from 0.01% to 5% by weight of compound of formula (I) or a stereoisomeric or polymorphic form thereof, based on the total weight of the composition.

19. Process for the preparation of a dermatological composition according to claims 9 to 16 by mixing a compound of formula (I) or a stereoisomeric or polymorphic form thereof, at least one dermatologically tolerable component and eventually further pharmaceutically active ingredients.

20. Process for the preparation of a dermatological composition according to claim 19 by mixing a compound of formula (I) or a stereoisomeric or polymorphic form thereof, at least one dermatologically tolerable component and eventually further pharmaceutically active ingredients, wherein the composition includes from 0.01 % to 5% by weight of compound of formula (I) or a stereoisomeric or polymorphic form thereof, based on the total weight of the composition.
